# EUROPÄISCHE PATENTANMELDUNG

(11) **EP 0 885 601 A2**
(43) Veröffentlichungstag der Anmeldung: **23.12.1998**
(21) Anmeldenummer: 98110768.3
(22) Anmeldetag: 12.06.1998
(51) Int. Cl.: A61F 7/00

(54) **Kühlelement**

(30) Priorität: 17.06.1997 DE 29710493 U; 10.07.1997 DE 29712140 U
(71) Anmelder: Sievers, Gabriele, 24116 Kiel (DE); Sievers, Hans-Jürgen, 24105 Kiel (DE); Bennecke, Jörg, 24116 Kiel (DE)
(72) Erfinder: Sievers, Gabriele, 24116 Kiel (DE); Sievers, Hans-Jürgen, 24105 Kiel (DE); Bennecke, Jörg, 24116 Kiel (DE)
(74) Vertreter: Biehl, Christian, Dipl.-Phys.

(57) **Zusammenfassung**

Kühlelement, gekennzeichnet durch eine Außenhülle aus einem wasserdampfdurchlässigen Material, die mit wenigstens einer ein Hydrogel enthaltenden Kühlkammer versehen ist, wobei ein als Band ausgeführter, aus wenigstens wasserdampfdurchlässigen Material gebildeten Streifen mit einer Kühlkammer oder eine flaschenmantelförmige Ausbildung mit einer Vielzahl von mit Versteppungen getrennten Kühlkammern, und z.B. einem im Bodenbereich eingelassenen, zusammenhaltenden Gummizug vorgeschlagen wird.

## Beschreibung

Die Erfindung betrifft ein Kühlelement, wie sie als Kühlauflage, beispielsweise als Stirnband, Handgelenkband, Halsband oder auch als Auflage auf die Augen Verwendung finden, oder auch als Hülle um beispielsweise Flaschen verwendet werden.

Bisher werden Kühlauflagen entweder als feuchte Kompressen oder als in Wasser undurchlässigen Taschen gehaltene Flüssigkeit oder sogar Eiswürfel, die durch vorheriges Kühlen, beispielsweise in einem Kühlschrank abgekühlt wurden, zum Kühlen benutzt.

Bei der ersten Möglichkeit nach dem Stand der Technik ist jedoch das feuchte Äußere der Kompressen unerwünscht, da dadurch Kleidungsstücke angefeuchtet werden und ggf. sogar ihre Farbe verlieren können.

Im zweiten Fall, den vorgekühlten "Kühl-Akkus" ist es zum einen schwierig, die gekühlten Kühlauflagen immer dann vorzuhalten, wenn Bedarf an ihnen besteht, zum anderen können diese Kühlauflagen ihre bestimmungsgemäße Aufgabe nicht lange und nicht gleichmäßig erfüllen und sind vergleichsweise schwer.

Der Erfindung lag daher die Aufgabe zugrunde, ein Kühlelement zu schaffen, die nicht feuchtet und über einen langen Zeitraum eine angenehme Kühle, insbesondere auf die Haut eines Benutzers, aufbringen kann, wobei die Kühlauflage noch leicht ist und unabhängig von Kühlschränken oder dergleichen über möglichst lange Zeit mit möglichst einfachen Mitteln betreibbar benutzt werden kann.

Erfindungsgemäß wird die Aufgabe durch die Merkmale des Anspruches 1 gelöst. Die Unteransprüche geben vorteilhafte Ausführungsformen der Erfindung wieder.

Insbesondere ist vorteilhaft, daß durch Einfüllen eines Hydrogels, eines im feuchten Zustand ('Lyogel') gelbildenden Materials, das im trockenen Zustand eine salzartige Struktur, ein sogenanntes Xerogel bildet, in Taschen eingebracht ist, die in der streifenförmigen Kühlauflage vorgesehen sind. Als Hydrogel wird dann bevorzugt ein solches ausgewühlt, daß in kleinen Brocken in einer Tasche verteilbar ist, aber nicht so flüssig ist, daß es durch die Schwerkraft 'fließt'. Eine aus solchen ca. 1-4 mm großen Partikeln bestehende Füllung läßt sich mit der Hand durch Glattstreichen egalisieren und behält dann ihre Form im wesentlichen bei.

Die Endbereiche der streifenförmigen Kühlauflage dienen dann entweder mit einem Klettverschluß zu Befestigung beispielsweise als Stirnband oder Armband, oder können auch bei einem Kühlhalsband einfach locker vor dem Hals verschlungen werden.

Vorteilhaft bei der Ausbildung der streifenförmigen Kühlauflage aus einem textilen Material, das wenigstens in trockenen Zustand wasserdampfdurchlässig ist, ist es, daß nun das Xerogel ganz kontinuierlich und über einen Zeitraum von z. B. zwei Tagen Wasserdampf abgeben kann, um eine Kühlwirkung zu erzeugen. Dabei werden, insbesondere wenn das textile Material Mikrofasern sind, die textilen Außenbereiche des Bandes bereits trocken sein.

Dennoch kann bei Verwendung von Mikrofasern das Hydrogel durch einfaches Eintauchen der gesamten Kühlauflage in Wasser befeuchtet werden. Gegebenenfalls überschüssiges Wasser wird dann bei der Entnahme aus dem Wasser heraus schnell abgegeben und nur noch das im Gel gebundene Wasser wird langsam über die nächsten beispielsweise zwei Tage abgegeben, wobei dies nicht ausreicht, daß die Mikrofaser feucht zu belassen.

Es ist auch denkbar, jedoch bei Mikrofasern nicht nötig, eine Seite der Kühlauflage mit einem wasserundurchlässigen Material und nur beispielsweise die Außenseite eines Stirnbandes mit Wasser bzw. wasserdampfdurchlässigem Material auszubilden.

Da handelsübliche Hydrogele eine körnige reisähnliche Struktur aufweisen, können sie in einer nicht weiter unterteilten Kammer der Kühlauflage durch einfaches "Schieben" zwischen zwei Fingern verteilt werden und sich der Körperkontur so gut anpassen.

Vorteilhaft bei dem einen Ausführungsbeispiel einer streifenförmigen Kühlauflage ist, daß allein durch Einlegen in kaltes oder maximal lauwarmes Wasser ein langfristiger Kühleffekt beginnen kann, der sich je nach Außentemperatur (stärkere Verdunstung) auch entsprechend stark den Umweltgegebenheiten anpaßt. Das Hydrogel verbraucht sich dabei nicht und kann weder im feuchten noch im trockenen Zustand in die Mikrofasern des textilen Materials einwandern, so daß es sicher in der Tasche gehalten ist.

Für Kühlhüllen oder Kühlbehälter werden bisher neben energieverbrauchenden aktiven Kühleinrichtungen lediglich isolierende Materialien in Kühlbehältern verwandt. Die ohne solche aktiven Kühleinrichtungen bekannten "Kühltaschen" erzielen ihre Kühlwirkung daneben dadurch, daß sogenannte Kühlakkus, meist mit Wasser gefüllte, in einer Gefriertruhe oder dergleichen auf entsprechende Minus-Grade gebrachte Behälter neben den kühl zu haltenden Gütern in die Kühltaschen eingebracht werden.

Solche Kühlung ist zu Beginn sehr stark, später dann sehr schwach und leidet unter dem Mangel, daß sie nicht unabhängig von primär eingesetzten Kühlschränken möglich ist.

Insbesondere ist es vorteilhaft, daß durch Einfüllen eines Hydrogels, eines im feuchten Zustand ("Lyogel") gelbildenden Materials, das im trockenen Zustand eine salzartige Struktur ein sogenanntes "Xerogel" bildet, eine im trockenen Zustand leichte Tasche vorhanden ist, die durch einfaches Befeuchten Verdunstungskälte an den Inhalt weitergeben kann. Als besonders geeignet, weil es nicht in die Faserstruktur einwandert, hat sich ein im xerogelen Zustand körnige Kristalle ausbildendes (CONH₂-COOR)ₙ (R = ein Alkalimetall wie z.B. K, Na) erwiesen, das im lyogelen Zustand zumindest bei Anwesenheit von SiO₂ kleine in den Kühlkammern verschiebbare Brocken ausbildet.

Im Gegensatz zu bisher bekannten wasserspeichernden und verdunstungskälteproduzierenden Einrichtungen, wie beispielsweise Tonkühlern für Weinflaschen, feuchtet eine mit Mikrofaser umschlossene Gelfüllung nach dem zweiten Ausführungsbeispiel nicht durch, sondern sie trocknet nach kurzem Eintauchen in Wasser durch noch bestehende Wasseraufnahmefähigkeit der Füllung unmittelbar, um dann lediglich Wasser im nicht feuchten, sondern dampfförmigen Zustand durchzulassen. Die Taschen oder Beutel nach der Erfindung sind leicht und nehmen wenig Platz ein.

Um Wasser in die Kühlkammern einzubringen, kann in einer Ausführung ein schmaler Bereich an der inneren Oberkante nicht aus Microfaser sondern aus normaler Faser vorgesehen werden, durch die beim Eintauchen des Kühlbehälters in Wasser dieses hindurchtreten kann.

Durch die Vorsehung mehrerer ringförmiger, um einen zylindrischen Hohlraum angeordneten Kühlkammern bei einer Kühlhülle kann bei minimaler Außenfläche ein größtmöglicher Raum umgeben werden und das Hydrogel gleichmäßig gehalten werden. Insbesondere bei einer Ausbildung als Kühltasche kann eine innere kastenartige Behälterstruktur eine wasserdichte Eingabemöglichkeit beispielsweise für lose Lebensmittel oder Eiswürfel bieten.

Weitere Merkmale und Vorteile der Erfindung ergeben sich aus nachfolgender Beschreibung eines bevorzugten Ausführungsbeispiels. Dabei zeigt:
- Fig. 1: eine streifenförmige Kühlauflage in Draufsicht,
- Fig. 2: eine streifenförmige Kühlauflage wie in Fig. 1 im Querschnitt,
- Fig. 3: eine streifenförmige Kühlauflage als Halsband,
- Fig. 4: eine streifenförmige Kühlauflage als Gesichtsmaske,
- Fig. 5: einen flaschenmantelförmigen mit Versteppungen in eine Vielzahl von Kühlkammern aufgeteilten Kühlbehälter, und
- Fig. 6: einen Kühlbeutel mit drei Kühlkammern.

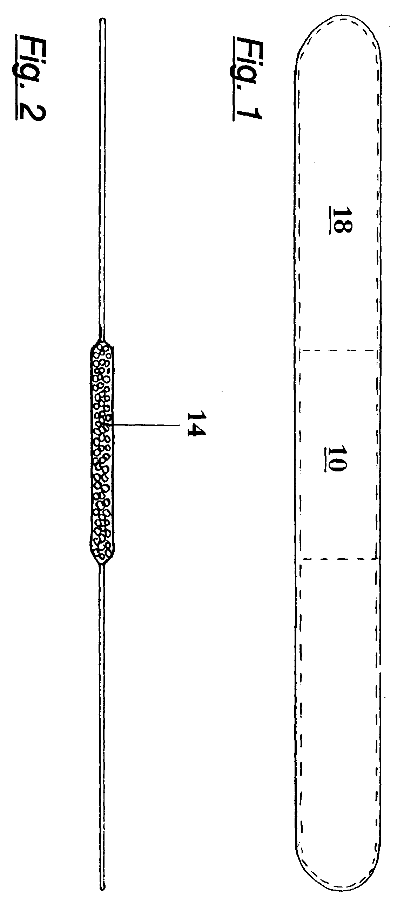

Die in der Fig. 1 dargestellte Kühlauflage weist eine mittige, im wesentlichen die gesamte Breitenerstreckung der Kühlauflage einnehmende Tasche 10 auf, an die sich in den Endbereichen textiles, nicht gefülltes Streifenmaterial 18 anschließt. In der Tasche 10 ist, wie in der Fig. 2 im Querschnitt zu erkennen, ein Hydrogel in Form von kleinen reisartigen Partikeln 14 eingebracht, die im trocknen Zustand salzähnlich als Xerogel vorhanden sind und bei Befeuchten innerhalb kürzester Zeit zu einem Lyogel (kleinen gallertartigen Partikeln) durch Aufnahme von Wasser als Dispersionsmittel umwandeln.

Durch langsame Abgabe des Wassers durch die Mikrofasern, die das Gewebe Wasserdampf-durchlässig machen, bildet sich ein Kühleffekt, der noch dadurch verstärkt werden kann, daß man kühles Wasser zum Dispergieren des Hydrogels verwendet.

Dadurch, daß Mikrofasern auf beiden Seiten des Kühlbandes, beispielsweise eines Stirnbandes, vorhanden ist, kann auch eine gute Wärmebrücke zu den gekühlten Körperpartien hergestellt werden, so daß mit einem schnellen Einfluß auf die Blutzirkulation in erwünschter Weise zu rechnen ist.

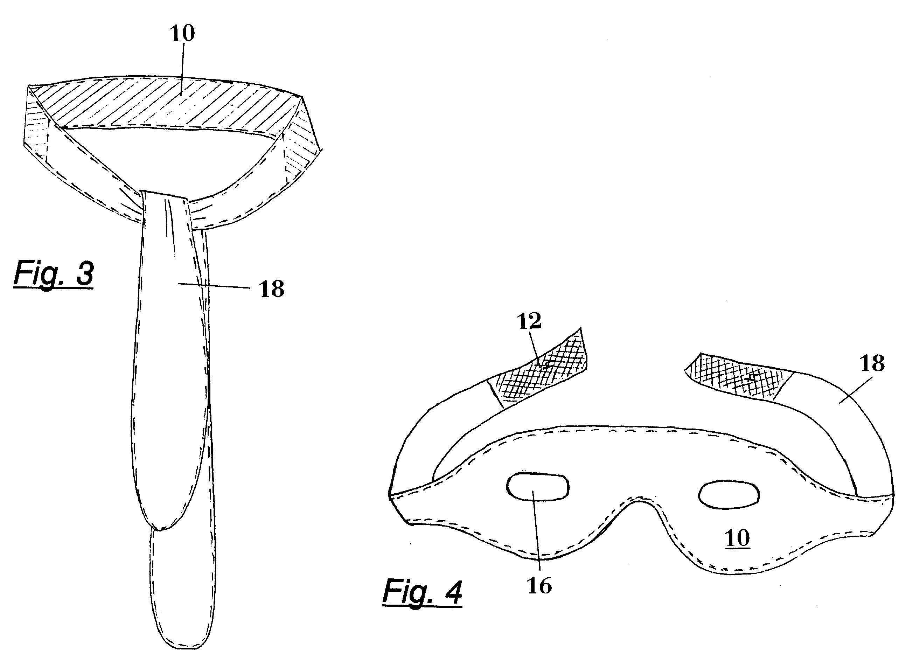

In der Fig. 3 ist in einer als Kühlhalsband mit wesentlich längerer Tasche 10 ausgebildeten Ausführung die Möglichkeit dargestellt, die Hals- und Nackenpartie zu kühlen.

Nicht dargestellt, jedoch sehr einfach herstellbar, sind Armgelenk, Kühlauflagen, bei denen, wie auch in der Fig. 4 dargestellt, am Ende ein Klettverschluß zum größenverstellbaren Zusammenschluß der Enden vorhanden ist. Es ist jedoch auch denkbar, einen zusätzlichen Gummizug in einen geschlossenen Ring der streifenförmigen Kühlauflage einzubringen.

Fig. 4 ist schließlich eine weitere vorgeschlagene Form der Kühlauflage, eine Gesichtsmaske mit Öffnungen für die Augen 16 und einer randseitigen Aussparung für die Nase zu entnehmen, die in angenehmer Weise die Augen- und Wangenpartie kühlt.

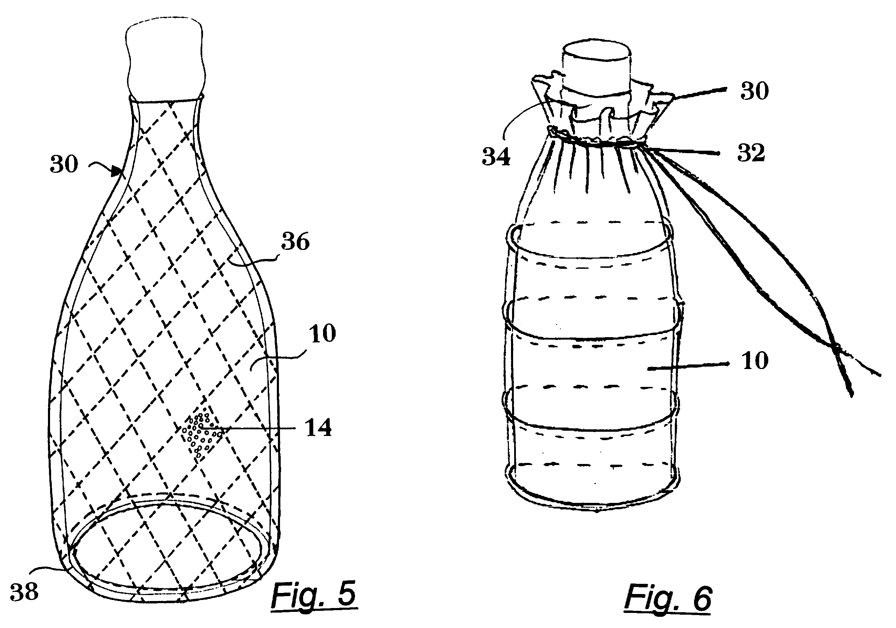

Bei einer Verwendung des Kühlelements als Kühlhülle wie in Fig. 5 und Fig. 6 dargestellt, enthält das Gewebe der Außenhülle des Kühlbehälters vorteilhafterweise eine eingelegten Lage aus Vliesstoff, auf den die körnigen Hydrogelbrocken im xerogelen Zustand, bevorzugt zusätzlich mit einer Bügeleisen erhitzt, aufgebracht werden. Die in diesem Zustand leicht klebrigen Kristalle werden sich in dem flauschigen Gewebe verfangen und so für die Zukunft immobilisiert. Erst anschließend erfolgt ein Belegen beider Seiten mit einem Gewebestoff, der wasserdampfdurchlässig ist, in breiten Bahnen und ein Versteppen, um gleichzeitig das Volumen der Außenhülle zu steigern.

Der Vliesstoff sorgt dabei nicht nur für die Verankerung der Gelbrocken, so daß diese sich nicht im unteren Abschnitt der Kammer zu sammeln beginnen, sondern verteilt eindringendes Wasser und schafft eine Belüftung im trocknenden Zustand, die die Gelbrocken gleichmäßig erreicht.

Der in der Fig. 6 dargestellte Kühlbeutel 30 ist am oberen Ende mit einem Zugband 32 versehen, um den Beutel beispielsweise um Flaschenhälse 34 herumlegen zu können und so sicher an dem zu kühlenden Gegenstand zu halten. Drei ringförmig sich um den zu kühlenden Gegenstand Legende Kühlkammern 10 und eine Kühlkammer am Boden sind mit Hydrogel gefüllt und jeweils durch Nähte voneinander separiert.

Bei Nichtgebrauch können die Kühlkammern 10 in sich eingefaltet werden, so daß der Beutel insbesondere, wenn das Hydrogel in einem trockenen Zustand ist, nur geringfügig Platz beansprucht. Die Kühlkammern sind als jeweils einige Zentimeter breite umlaufende Streifen in der Hülle ausgebildet, die durch Nähte voneinander getrennt sind, um jeweils Gel in sich aufzunehmen.

Die in der Fig. 5 vorhandenen abgesteppten kleineren Kammern halten die Gelpartikel 14 besser auf der gesamten Oberfläche und kühlen die Flasche insbesondere im Halsbereich besser, was beispielsweise für Champagner wichtig ist. Außer ästhetisch schönerer Form ist es möglich Metallfolien zur Reflektion von äußerer (Infrarot-) Strahlung aufzubringen, die bei einem Beutel wie in Fig. 6 leicht zu sehr geknickt würden.

Durch die Vorbereitung breiter Bahnen des Vliesstoffes kann durch Herausschneiden jeder gewünschten Form eine einfache kostengünstige Anpassung der Kühlbehälter an beliebige Formen erfolgen. Dabei kann wie in Fig. 5 ebenfalls dargestellt noch durch die Einbringen von Befestigungseinrichtungen, wie des Gummizugs 38 am Boden der Flasche eine individuelle Paßform erzeugt werden.

## Patentansprüche

1. Kühlelement, gekennzeichnet durch eine Außenhülle (30) aus einem wasserdampfdurchlässigen Material, die mit wenigstens einer ein Hydrogel enthaltenden Kühlkammer (10) versehen ist.

2. Kühlelement nach Anspruch 1, gekennzeichnet durch einen als Band ausgeführten, aus wenigstens wasserdampfdurchlässigen Material gebildeten Streifen mit einer Kühlkammer (10), in der eine Hydrogelfüllung (14) enthalten ist.

3. Kühlelement nach Anspruch 2, dadurch gekennzeichnet, daß das wasserdampfdurchlässige Material aus Mikrofasern besteht, die wenigstens im trockenen Zustand wenigstens wasserdampfdurchlässig sind.

4. Kühlelement nach einem der vorangehenden Ansprüche, dadurch gekennzeichnet, daß zur Ausbildung eines Stirnbandes die Enden mit den Elementen eines Klettverschlusses belegt sind.

5. Kühlelement nach einem der vorangehenden Ansprüche, dadurch gekennzeichnet, daß die Innenseite der Kühlkammer(n) (12) undurchlässig für Wasser im flüssigen Zustand ist.

6. Kühlelement nach einem der vorangehenden Ansprüche, dadurch gekennzeichnet, daß ein im xerogelen Zustand körnige Kristalle ausbildendes (CONH₂-COOR)ₙ (R=Alkalimetall) als Hydrogel verwandt wird, das im lyogelen Zustand kleine Gel-Brocken (14) bildet.

7. Kühlelement nach einem der vorangehenden Ansprüche, dadurch gekennzeichnet, daß zur Ausbildung einer einen Kühlbehälter bildenden Kühlhülle ein dreilagiges Gewebe verwandt wird, dessen mittlere Lage aus einem Vliesstoffes mit dem Hydrogel beladen ist, und die Außenlagen aus Hydrogel-undurchlässigem Gewebe bestehen.

8. Kühlelement nach Anspruch 7, gekennzeichnet durch eine flaschenmantelförmige Ausbildung mit einer Vielzahl von mit Versteppungen (36) getrennten Kühlkammern (10), und einem im Bodenbereich eingelassenen, zusammenhaltenden Gummizug (38).

9. Kühlelement nach Anspruch 7, gekennzeichnet durch eine beutelartige Ausbildung mit einer Mehrzahl von ringförmigen übereinander angeordneten Kühlkammern (10), die einen zylindrischen Innenraum umschließen.

10. Kühlelement nach Anspruch 7, gekennzeichnet durch eine taschenartige Ausbildung in der ein kastenförmiges, wasserundurchlässiges Stabilisierungselement vorgesehen ist, an dem außen an den Seitenflächen und einem Deckelelement angebrachte Kühlkammern (12) angeordnet sind.
